# EUROPEAN PATENT SPECIFICATION

(11) **EP 2 574 931 B1**
(45) Date of publication and mention of the grant of the patent: **22.03.2017**
(21) Application number: 11183276.2
(22) Date of filing: 29.09.2011
(51) Int. Cl.: G01N 21/64

(54) **Dry composition comprising a control dye**
Trockenzusammensetzung mit einem Steuerfarbstoff
Composition sèche comprenant un colorant de contrôle

(43) Date of publication of application: 03.04.2013
(73) Proprietor: Qiagen GmbH, 40724 Hilden (DE)
(72) Inventor: Jeziorski, Markus, 40764 Langenfeld (DE); Wende, Andy, 40724 Hilden (DE)
(74) Representative: Roth, Carla

(56) References cited:
- EP-A1- 2 239 338
- WO-A1-2010/001162
- WO-A2-01/92569
- WO-A2-2006/069023
- DE-A1-102007 045 474
- US-A- 5 203 385

## Description

The present disclosure pertains to dry compositions in particular freeze-dried compositions comprising at least one reagent and at least one control dye, in particular a fluorescent dye, and methods of reconstituting respective dry compositions for performing chemical and/or biotechnological methods involving the at least one reagent comprised in the dry composition. The control dye allows to monitor and control the reconstitution process thereby avoiding errors in the chemical and/or biotechnological method due to variations/errors in the reconstitution process of the reagent(s) comprised in the dry composition. Furthermore, the control dye allows to analyse residual moisture in the dry composition thereby allowing to monitor and control the quality of the production process and the intactness of the dry composition after storage.

Dry compositions of reagents are widely used in chemical and/or biotechnological methods, in particular in amplification reactions such as e.g. the polymerase chain reaction. Respective dry compositions usually comprise one or more or even all reagents necessary for the chemical and/or biotechnological method such as e.g. enzymes, buffers, salts, oligonucleotides and the like. The use of respective dry compositions, in particular freeze-dried compositions, has the advantage that the dry compositions are stable during storage and if all reagents necessary for the intended reaction are comprised therein, there is no need for the customer to prepare the reaction mix by adding, respectively mixing the reaction compounds. Rather, the customer only needs to add a pre-determined amount of liquid for reconstituting the dry composition in order to obtain the prepared reaction mix that can be e.g. contacted with a sample in case of an amplification reaction. Of course, the customer may optionally also add at least one further reagent such as e.g. specific reaction primers in case of an amplification reaction. Respective freeze-dried compositions are in particularly used when processing a large number of samples using the same type of reaction mix, respectively reaction conditions, respectively when performing a large number of reactions and in particular in automated applications. One large field of application is the analysis of samples, e.g. for diagnostic or therapeutic applications. Using customized, prepared freeze-dried reagents reduces the risk of errors that result from the set up of the reaction conditions, respectively the mixture of the reagents necessary for the intended chemical and/or biotechnological method.

The preparation of respective freeze-dried compositions suitable for performing chemical and/or biotechnological methods as well as their use in different reactions in particular for performing amplification reactions are well known in the prior art. The preparation and stabilisation of respective freeze-dried composition, respectively the components contained therein, such as biological products, in particular enzymes, such as for example polymerases is also described in the prior art (see for example WO 01/92569, WO 2010/001162, US 2010/0068716 and US 2010/0159529). Therein, freeze-dried compositions are described that comprise reagents for PCR reactions and may include a fluorescent reporter in order to detect the PCR product and/or to follow the PCR process. WO 2010/001162 A1 and WO 01/92569 A2 disclose dry compositions comprising a dye. DE 10 2007 045474 A1 discusses the use of magnetic particles for selective enrichment or separation of biological material from a solution.

When using respective dry compositions for performing chemical and/or biotechnological methods, several problems may arise. First of all it must be ensured that the dry composition does not comprise residual moisture exceeding a desired/allowed level. Residual moisture is the low level of water, usually in the range of less than 5%, usually in the range of 1-3%, that remains in a freeze-dried product after the freeze-drying (e.g. vacuum sublimation process) is complete. The residual moisture content in the freeze-dried product is an important quality feature, because it affects the potency of the product, its long-term stability and its shelf-life (see e.g. Freeze-Drying/Lyophilization of Pharmaceutical and Biological Products, Second Edition, page 350 et seq). Optimal residual moisture limits are set for each freeze-dried biological products on a case-by-case basis. Residual moisture may not only influence the stability, but also the potency. Thus, elevated amounts of residual moisture can be detrimental for the quality of the dry composition, in particular if the dry composition comprises an biological compound such as a protein such as for example a polymerase because the activity of the enzyme can decrease if the composition is not sufficiently dry and comprises e.g. a residual moisture of 5% or more. Furthermore, it is also important to ensure that the dry composition was not compromised during storage, and for example, has not drawn moisture during the storage process because the residual moisture resulting from respective improper storage could affect again the quality of the freeze-dried reagents such as e.g. proteins, in particular enzymes such as polymerases. Respectively compromised samples can have the effect that the intended reaction is erroneous.

A further issue when using a dry composition in a chemical and/or biotechnological method is to control that the reconstitution process is performed properly. Here, different aspects are of importance. First of all, it is important to ensure that a pre-determined amount of liquid is added to the dry composition for the reconstitution process. Second, it is important to ensure that the overall reconstitution process is effective and that the comprised reagents are effectively dissolved during reconstitution because an incomplete reconstitution can negatively affect the subsequent chemical and/or biotechnological method as e.g. the reagents are not provided in the intended concentration in the reaction mixture.

For controlling the amount of fluid that is added to a reaction, it is known in the prior art to use a fluid transfer control system wherein a solution comprising a control dye is used in order to determine that the fluid transfer was successful (see e.g. EP 2 239 338). However, respective methods also have their drawback as this system does not allow to analyse whether the reconstitution processes was successful.

Therefore, it was an objective of the present invention to provide a technology that allows to monitor and/or to control the reconstitution process of a dry composition suitable for performing a chemical and/or biotechnological method. Furthermore, it was an objective of the present invention to provide a technology that allows to detect residual moisture in said dry composition suitable for performing a chemical and/or biotechnological method.

### SUMMARY OF THE INVENTION

The present invention can be defined by the appending claims.

The present invention is based on the finding that incorporating at least one control dye, preferably a fluorescent dye, into a dry composition of reagents allows to analyse the reconstitution process by measuring the intensity of the control dye, preferably its fluorescence, during and/or after reconstitution. Furthermore, the at least one control dye, which preferably is a fluorescent dye, that is incorporated in the dry composition according to the present disclosure can also be used in order to analyse the amount of residual moisture contained in the dry composition by measuring the intensity of the control dye, preferably its fluorescence. Therefore, the present invention provides an elegant method for analysing and controlling essential factors that are important for the quality of the subsequent chemical and/or biotechnological method.

According to a first aspect of the present invention, a method of reconstituting a dry composition comprising at least one reagent suitable for a chemical and/or biotechnological method is provided, said method comprising the following steps:
a) obtaining a dry composition comprising at least one reagent and at least one control dye, preferably a fluorescent dye;
b) reconstituting the dry composition;
c) analysing the reconstitution process by measuring at least one property of the control dye, preferably its fluorescence, during and/or after reconstitution.

The incorporation of the control dye, which preferably is a fluorescent dye, into the dry composition has the advantage that the reconstitution process can be efficiently analysed and thus controlled. The reconstitution process is analysed by measuring at least one property of the control dye, preferably its fluorescence, during and/or after reconstitution. For reconstitution, a liquid can be added. A "liquid" as used herein in particular refers to any flowable composition. Suitable liquids include but are not limited to water, compositions comprising water, buffers, suspensions, aqueous compositions, solutions and the like. Thus, the liquid may also comprise solid components. Preferably, a pre-determined amount of liquid is added for reconstitution to ensure that after reconstitution, a composition is provided that comprises the at least one reagent in the desired concentration.

Based upon the measured intensity of the control dye, e.g. its fluorescence, it is possible to determine that liquid was added to the dry composition for reconstitution and furthermore, it can be controlled that a desired pre-determined amount of liquid was added. Additionally, the method according to the present invention allows to analyse whether the reconstitution process was successful, i.e. that the reagent(s) comprised in the dry composition is/are homogenously dispersed in the reconstituted solution.

The present invention also provides a chemical and/or biotechnological method comprising the following steps:
a) obtaining a dry composition comprising at least one reagent for performing the chemical and/or biotechnological method and at least one control dye, preferably a fluorescent dye;
b) reconstituting the dry composition;
c) analysing the reconstitution process by measuring at least one property of the control dye, preferably its fluorescence, during and/or after reconstitution;
d) optionally adding further reagents and/or additives to the reconstituted dry composition;
e) performing the chemical and/or biotechnological method.

Controlling the reconstitution process based upon the control dye that is incorporated in the dry composition according to the present invention has considerable advantages for the quality of the subsequent chemical and/or biotechnological method as discussed above and below. The reconstitution process is analysed by measuring at least one property of the control dye, preferably its fluorescence, during and/or after reconstitution.

The present invention also pertains to the use of at least one fluorescent dye in a dry composition comprising at least one reagent for performing a chemical and/or biotechnological method for analysing residual moisture in said dry composition.

Also disclosed is the use of at least one control dye, preferably a fluorescent dye, in a dry composition comprising at least one reagent suitable for performing a chemical and/or biotechnological method for analysing and/or monitoring the reconstitution process of the dry composition. As discussed above, measuring e.g. the fluorescence of the control dye allows to analyse that the reconstitution of the dry composition was successful. Thus, the teachings allow to ensure that the reaction conditions for performing the chemical and/or biotechnological method are optimal, respectively within an acceptable range and accordingly, are not negatively affected by the reconstitution process.

The present invention also pertains to the use of at least one control dye, which is a fluorescent dye, that is incorporated into a dry composition comprising at least one reagent suitable for performing a chemical and/or biotechnological method for analysing residual moisture in the dry composition. Determining e.g. the fluorescence of the control dye allows to analyse the amount of residual moisture contained in the dry composition. Thereby, it is possible to analyse, respectively control that the residual moisture within the dry composition does not exceed the allowed, respectively desired threshold. E.g. the detection of the fluorescence of the control dye within the dry composition allows to analyse the quality of the dry composition after storage, e.g. prior to the reconstitution of the dry composition. This is important because this allows to determine that the dry composition was not compromised during improper storage by adsorbing moisture, as this could be detrimental for the components comprised in the dry composition, in particular biochemical components, such as e.g. reaction enzymes. It is important to be able to determine whether the dry composition was respectively compromised, as this could be detrimental for the subsequent chemical and/or biotechnological method that is supposed to be performed with the reconstituted composition.

Also disclosed is a dry composition suitable for performing a chemical and/or biotechnological method comprising at least one reagent, preferably a biological product such as a protein, preferably an enzyme, and at least one control dye, in particular a fluorescent dye for analyzing and/or monitoring the subsequent reconstitution process and/or for analysing residual moisture contained in the dry composition.

Other objects, features, advantages and aspects of the present application will become apparent to those skilled in the art from the following description and appended claims.

### DETAILED DESCRIPTION OF THE INVENTION

The present invention can be defined by the appending claims.

The present invention is based on the finding that incorporating a control dye, preferably a fluorescent dye, into a dry composition comprising at least one reagent suitable for performing a chemical and/or biotechnological method can be used for monitoring the quality of the dry composition with respect to residual moisture and furthermore, can be used for monitoring the reconstitution process of the dry composition. Thereby, the reliability of the subsequent chemical and/or biotechnological method can be greatly improved because deviations attributable to errors/variations in the reconstitution process and/or attributable to residual moisture within the dry composition can be securely identified.

According to a first aspect of the present invention, a method of reconstituting a dry composition comprising at least one reagent suitable for a chemical and/or biotechnological method is provided, said method comprising the following steps:
a) obtaining a dry composition comprising at least one reagent and at least one control dye, preferably a fluorescent dye;
b) reconstituting the dry composition;
c) analyzing the reconstitution process by measuring at least one property of the control dye, preferably its fluorescence, during and/or after reconstitution.

Also disclosed is a method of reconstituting a dry composition comprising at least one reagent suitable for a chemical and/or biotechnological method is provided, said method comprising the following steps:
a) obtaining a dry composition comprising at least one reagent and at least one control dye, preferably a fluorescent dye;
b) reconstituting the dry composition;
c) analyzing the reconstitution process by measuring at least one property of the control dye, preferably its fluorescence, prior, during and/or after reconstitution.

As discussed above, the incorporation of the control dye, which preferably is a fluorescent dye, into the dry composition has the advantage that the reconstitution process can be efficiently analysed and thus controlled. Based upon the measured intensity of the control dye, e.g. its fluorescence, it is possible to determine that liquid was added to the dry composition for reconstitution. Furthermore, it can be controlled that the intended pre-determined amount of liquid was added for reconstitution. Additionally, the method according to the present invention allows to analyse whether the reconstitution process was successful, i.e. that the reconstituted reagent(s) is/are homogenously dispersed in the reconstituted solution. Performing a respective reconstitution control is important in order to ensure that the subsequent chemical and/or biotechnological method that involves the use of the reconstituted dry composition can be performed properly. Thereby, potential deviations in the outcome of the chemical and/or biotechnological method that are attributable to errors/deviations in the reconstituted reaction composition that could result e.g. from an improper reconstitution process can be identified and avoided. Hence, sources of errors can be identified and optionally, be eliminated. Thus, the present invention provides a major improvement of existing processes, as it allows to control that the reconstitution process of the dry composition was successful and accordingly, is within acceptable ranges/deviations what is essential for the performance of the subsequent chemical and/or biotechnological method.

Several control dyes can be used in the methods according the present invention. Suitable control dyes include, but are not limited to fluorescent dyes, light absorbing dyes (e.g. UV, VIS, infrared). Preferably, a fluorescent dye is used as control reagent. Using a fluorescent dye has considerable advantages because the fluorescence can be easily measured and thus be determined using an appropriate detector. Furthermore, when using the dry composition for preparing the reaction mix for performing a polymerase chain reaction (PCR), in particular a PCR reaction which involves the use of fluorescently labelled primers and probes, such as a real-time PCR, a fluorescence detector is usually already incorporated into the PCR machine so that no additional equipment is needed in order to determine the fluorescence of the comprised sample either prior, during and/or after reconstitution. In order to detect the control dye a compatible detector is used. Respective detectors are well known to the skilled person and thus, need no detailed description. Examples include, but are not limited to QIAGEN Rotor-Gene Q, QIAGEN ESElog, Roche LightCycler, spektrometers,photomultipliers and UV/VIS spectrophotometers.

According to one embodiment, the fluorescent dye is selected from the group consisting of rhodamine dyes, SYBR green, pico green, auramine, benzanthrone, coelenterazine, coumarine, benzimide, DAPI, ethidium bromide, homidium bromide, euxanthic acid, firefly luciferin, fluoresceine, fluoresceine derivatives, carboxy fluorescein compounds, cyanine dyes, fluorescein isothiocyanate, perylene, 9,10-bis(phenylethynyl)anthracene, rubrene, stilbene, acridinium dyes, carbazol dyes, phenoxazine dyes, porphyrine dyes, polymethin dyes, BODIPY, Texas Red and TSQ.

According to one embodiment, the fluorescent dye that is used as control dye is no intercalating dye. When performing an amplification reaction it can be advantageous that no intercalating dye is used as control dye because the fluorescence intensity may depend on the DNA concentration which can result in imprecise results.

According to one embodiment, the dry composition comprises additional dyes, e.g. fluorescent dyes. Respective additional dyes can form part of one or more reagents comprised in the dry composition. E.g. fluorescent dyes can be used in order to label proteins, enzymes, in particular polymerases, oligonucleotides, e.g. primers and/or probes. Respectively labelled reaction reagents are e.g. used in amplification applications, such as PCR reactions, in particular real-time PCR or digital PCR. According to one embodiment, the fluorescent dye that is used as control dye differs from the fluorescent dye(s) incorporated into the dry composition as reaction compound or part thereof. Thereby, it can be ensured that the fluorescent compound that is used as control dye does not interfere with the subsequent chemical and/or biotechnological method. Furthermore, the fluorescence of fluorophor/quencher labelled oligonucleotides is dependent on the temperature. It is, however, also within the scope of the present invention that the control dye, which preferably is a fluorescent dye, of a labelled reaction compound comprised in the dry composition such as e.g. an enzyme or at least one oligonucleotide is used as control dye. This embodiment has the advantage that no additional compound needs to be added to provide the reconstitution control according to the present invention.

According to one embodiment, the control dye that is used for analysing the reconstitution process is not used for and/or is not capable for reporting a change in the amount of double stranded DNA in a polymerase chain reaction. According to one embodiment, the fluorescent compound that is used for analysing the reconstitution process is not coupled to an oligonucleotide, primer and/or probe that is incorporated in the dry composition as reaction compound. According to one embodiment, it is not coupled to a biological product, in particular an enzyme that is incorporated in the dry composition as reaction compound.

For reconstituting the dry composition a pre-determined volume of liquid is added to the dry composition which usually is also provided in a pre-determined amount, e.g. comprised, preferably prefilled in a container or reaction vessel. The reconstitution process can be assisted e.g. by pipetting up and down, stirring, shaking or vortexing. According to one embodiment, the reconstitution process respectively the mixture of the reagent(s) is assisted by using a magnet. Thus, according to one embodiment, a magnetic material is comprised in the dry, preferably the freeze-dried composition. Said magnetic material allows to stir and/or mix the composition during and/or after reconstitution by the aid of a magnet. Preferably, the magnetic material is incorporated into the composition before it is dried, preferably freeze-dried. Thereby, the magnetic material becomes incorporated into the dry composition and can assist the reconstitution process. A preferred method is e.g. described in DE102007045474. According to one embodiment, the dry composition comprises a multiplicity of magnetic particles as well as at least one magnetic and/or magnetizable central element, which preferably is configured in the shape of a rod, dumbbell and/or ellipsoid. The magnetic and/or magnetizable central element is larger than the multiplicity of the magnetic particles. Preferably, these elements are incorporated into the composition before it is dried, preferably freeze-dried. The composition that was contacted for reconstitution with a liquid comprises a multiplicity of the first magnetic particles as well as at least one central element, which preferably is configured in the shape of a rod, dumbbell and/or ellipsoid. The magnetic particles are distributed in the liquid, and subsequently accumulate on the at least one central element. This process is assisted by at least one external magnet, e.g. a permanent magnet or electromagnet, which is configured to interact with the at least one central element.

The magnetic particles may comprise or consist of a material selected from the group paramagnetic materials, superparamagnetic materials, ferromagnetic materials, ferrimagnetic materials and mixtures thereof.

According to one embodiment, a defined amount of the dry composition is reconstituted by adding a pre-defined amount of liquid and preferably, is mixed during and/or after reconstitution.

According to one embodiment, it is determined based upon the fluorescence of the control dye whether liquid was (at all) added to the dry composition. According to one embodiment, it is determined whether a pre-defined amount of liquid has been added to the dry composition i.e. that the dry composition was contacted with the intended liquid volume for reconstitution. E.g. it was found in experiments that a higher fluorescence signal is detected in samples wherein no liquid was added at all and/or wherein less than the pre-determined amount of liquid was added if a fluorescent control dye is used. However, depending on the used control dye also other changes in the fluorescence might be observed and accordingly, can be used in the method according to the present invention. Hence, differences in the fluorescence that arise if no liquid was added and/or if an insufficient volume of liquid was added for reconstitution are measurable and quantifiable thereby providing an efficient control that allows to monitor the reconstitution process. Thus, according to one embodiment it is determined whether the dry composition is and/or has been reconstituted in the desired amount of liquid by detecting the fluorescence during and/or after reconstitution. According to one embodiment, variations in the filling quantity of the reconstituted dry composition are determined by detecting the fluorescence during and/or after reconstitution. As discussed above, preferably, a higher fluorescence signal is detected if less amounts of liquid are used for reconstitution.

According to one embodiment, the homogeneity of the reconstituted dry composition is analysed by detecting the fluorescence of the control dye during and/or after reconstitution. It was found e.g. in experiments with fluorescent control dyes that a higher fluorescence signal is detected in a non-homogenously reconstituted composition, in particular at the bottom of the vessel, than in a homogenously reconstituted composition. Depending on the control dye used, also other changes might be observable. These differences in the fluorescence are measurable and quantifiable, thereby providing an efficient control that allows to monitor the reconstitution process.

According to one embodiment, the amount of reconstituted reagents is quantified by detecting the fluorescence during and/or after reconstitution.

According to one embodiment, the characteristic property of the control dye, preferably its fluorescence, is determined before the dry composition is reconstituted and hence, prior to reconstitution.

As discussed above, all these features are determinable either alone or in combination using the control dye that was incorporated into the dry composition.

According to one embodiment, the measured intensity of the control dye, e.g. its fluorescence, is compared to at least one internal control and/or at least one standard. E.g. a threshold value or a threshold range can be pre-defined for the respective dry composition that it should achieve after proper reconstitution. An error could be indicated if the pre-defined thresholds are not met. Alternatively and/or additionally, the measured values can be compared to a positive control. This allows to verify that the samples were properly reconstituted and accordingly, that the results of the chemical and/or biotechnological reaction are significant, or allows to detect samples wherein an error occurred during reconstitution (e.g. no or not sufficient liquid was added or the reconstituted sample was improperly mixed). It is within the scope of the present invention to accept certain deviations of the measured intensity, e.g. of about 15%, 10%, 5% or 2% (or more or less), depending on the intended chemical and/or biotechnological reaction. The respective sample can be marked e.g. as "control passed" or "control failed". E.g. a respective standard or threshold, respective threshold range can also be stored within the detector. A suitable standard or threshold is e.g. the approximate value that was achieved with said composition or a comparable composition before it was dried (preferably freeze-dried) wherein certain deviations of e.g. +/- 15%, +/-10%, preferably +/- 5% or +/- 2%, are acceptable. Therefore, according to one embodiment, the intensity of the control dye, preferably its fluorescence, is determined before the composition is dried and said value is used for defining a standard or threshold or threshold range.

The dry composition that is used in the method according to the present invention is a storable composition. Preferably, it is suitable for long-term storage. According to one embodiment, the dry composition is stable during storage for a time period of at least 3 months, at least 6 months, at least 10 months or at least 12 months. Preferably, the dry composition is stable for a time period of 3 to 18 months or 6 to 12 months.

According to one embodiment, the dry composition is a freeze-dried composition. Freeze-dried compositions are widely used for providing the reagents necessary for chemical and/or biotechnological methods in a storable form. Respective freeze-dried compositions are in particular used in the field of biotechnology, in order to provide reagents necessary for the intended biotechnological method in a prepared, preferably premixed and thus easy to use form. Respective freeze-dried compositions often comprise at least one biological product, e.g. selected from nucleic acids such as oligonucleotides, proteins, enzymes and the like. Methods for preparing respective freeze-dried compositions as well as suitable additives that stabilise the comprised reaction compositions, in particular biochemical components such as enzymes are well-known in the prior art (see e.g. Freeze-Drying/Lyophilization of Pharmaceutical and Biological Products, Second Edition, WO 01/92569, WO 2010/001162, US 2010/0068716 and US 2010/0159529) and thus, need no detailed description here. The control dye that is used according to the teachings of the present invention is added either before or during lyophilisation so that it is incorporated in the final dry composition. According to one embodiment, the dry composition that is reconstituted according to the method according to the present invention comprises at least some of the chemical and/or biochemical reagents necessary for conducting the intended chemical and/or biotechnological method. It may also comprise all of the necessary reagents. According to a preferred embodiment, the dry composition comprises at least some, preferably all, of the chemical and/or biochemical reagents necessary for conducting an amplification reaction, preferably a PCR reaction. As discussed above, respective dry compositions, in particular freeze-dried compositions, are widely used to provide the reagents necessary for the amplification in form of a so-called master mix, in a storable form. The dry composition only needs to be reconstituted when intending to perform the amplification reaction, optionally further reagents (if not all reagents were comprised in the dry composition) and the sample are added to form the final reaction mixture. According to a preferred embodiment, the dry composition comprises one or more, preferably all of the following chemical and/or biochemical reagents selected from the group consisting of a polymerase, a reaction buffer suitable for performing an amplification reaction and dNTPs. It may also comprise salts, primers and/or probes or additional enzymes. Furthermore, it may comprise a magnetic material that allows to support the reconstitution process by enabling the mixing of the composition during reconstitution by the aid of a magnet. After reconstitution, the resulting reconstituted composition is capable of effecting the intended effect, e.g. effecting amplification of a target nucleic acid in case a dry composition suitable for use in an amplification, e.g. a PCR reaction is provided. For the amplification reaction, a sample can be contacted with the reconstituted composition. Furthermore, optionally further compounds or additives can be added.

According to one embodiment, the dry composition comprises reagents suitable, respectively useful in a biotechnological method wherein said method is selected from the group consisting of amplification reactions, in particular polymerase chain reaction (PCR), reverse transcription polymerase chain reaction (RT-PCR), quantitative real time polymerase chain reaction (Q-PCR), digital PCR, nucleic acid sequencing reactions, restriction analysis, reverse transcription, NASBA, allele specific polymerase chain reaction, polymerase cycling assembly (PCA), asymmetric polymerase chain reaction, linear after the exponential polymerase chain reaction (LATE-PCR), helicase-dependent amplification (HDA), hot-start polymerase chain reaction, intersequence-specific polymerase chain reaction (ISSR), inverse polymerase chain reaction, ligation mediated polymerase chain reaction, methylation specific polymerase chain reaction (MSP), multiplex polymerase chain reaction, nested polymerase chain reaction, solid phase polymerase chain reaction, recombinase-polymerase amplification (RPA), or any combinations thereof. Respective dry compositions, in particular freeze-dried compositions are also applicable in other biotechnological and/or chemical reactions, in particular in the field of molecular biology.

According to one embodiment, the dry composition is comprised within a vessel. Suitable vessels include but are not limited to reaction vessels, containers, amplification vessels, multi-vessels such as e.g. multiwell plates and the like. Respective laboratory equipment is well known and thus, needs to detailed description here.

Furthermore, the present disclosure pertains to a method of using at least one control dye, preferably a fluorescent dye, in a dry composition comprising at least one reagent for a chemical and/or biotechnological method in order to analyse and/or monitor the reconstitution process. Thus, the present invention also provides a chemical and/or biotechnological method comprising the following steps:
a) obtaining a dry composition comprising at least one reagent and at least one control dye, preferably a fluorescent dye;
b) reconstituting the dry composition;
c) analysing the reconstitution process by measuring at least one property of the control dye, preferably its fluorescence, during and/or after reconstitution;
d) optionally adding further reagents and/or additives to the reconstituted composition;
e) performing the chemical and/or biotechnological method.

Disclosed is also a chemical and/or biotechnological method comprising the following steps:
a) obtaining a dry composition comprising at least one reagent and at least one control dye, preferably a fluorescent dye;
b) reconstituting the dry composition;
c) analysing the reconstitution process by measuring at least one property of the control dye, preferably its fluorescence, prior, during and/or after reconstitution;
d) optionally adding further reagents and/or additives to the reconstituted composition;
e) performing the chemical and/or biotechnological method.

Controlling the reconstitution process based upon the incorporated control dye has considerable advantages for the quality of the subsequent chemical and/or biotechnological method as is discussed above. Details of the dry composition, the control dye, the reconstitution process, the analysis of the reconstitution process, possible and preferred chemical and/or biotechnological methods and the advantages associated with the teachings of the present invention with respect to the achievable improvement of the subsequent chemical and/or biotechnological method are discussed above and also apply here. It is referred to the above disclosure.

Furthermore, the present disclosure pertains to the use of at least one control dye, preferably a fluorescent dye, present in a dry composition comprising at least one reagent suitable for a chemical and/or biotechnological method for analysing residual moisture in the dry composition. As discussed above, residual moisture in the dry composition can be a major problem because it can compromise the quality of the dry composition, respectively the at least one reagent comprised therein. This is particularly an issue for proteins comprised in the dry composition such as in particular enzymes such as polymerases that are regularly incorporated in dry compositions suitable for performing an amplification reaction such as e.g. a PCR reaction. It was found by the inventors that changes in the residual moisture comprised in the dry composition result in detectable, i.e. measurable changes in the intensity of the control dye, e.g. in its fluorescence when using a fluorescent dye as the preferred embodiment of a control dye. It was found in experiments that the fluorescence decreases if the residual moisture rises. However, also other changes in the measurable properties, in particular the fluorescence, can be used; this also depends on the used control dye.

Thus, the present invention also provides a method for analysing residual moisture in a dry composition of at least one reagent suitable for performing a chemical and/or biotechnological method, wherein at least one control dye, preferably a fluorescent dye, is incorporated into the dry composition and wherein the residual moisture in the dry composition is determined by measuring at least one property of the control dye, preferably its fluorescence. Thereby, it can be analysed and thus ensured e.g. that the residual moisture in the dry composition is within, preferably below a predetermined threshold limit, e.g. less than 15%, less than 10%, less than 5% or less than 2% e.g. when preparing a dry composition comprising an enzyme such as e.g. polymerase. Details of the dry composition and its preparation and the incorporated control dye are discussed above in conjunction with the method for reconstituting a dry composition and also apply here. It is referred to the above disclosure.

Furthermore, disclosed is a dry composition comprising at least one reagent for a chemical and/or biotechnological method comprising at least one fluorescent dye for analyzing and/or monitoring the reconstitution process and/or for analysing residual moisture in the dry composition. Details of the dry composition, its constituents and its preparation as well as on the incorporated control dye and its uses are discussed above and also apply here. It is referred to the above disclosure.

The present invention will now be illustrated by the following non-limiting examples.

### BRIEF DESCRIPTION OF THE DRAWINGS

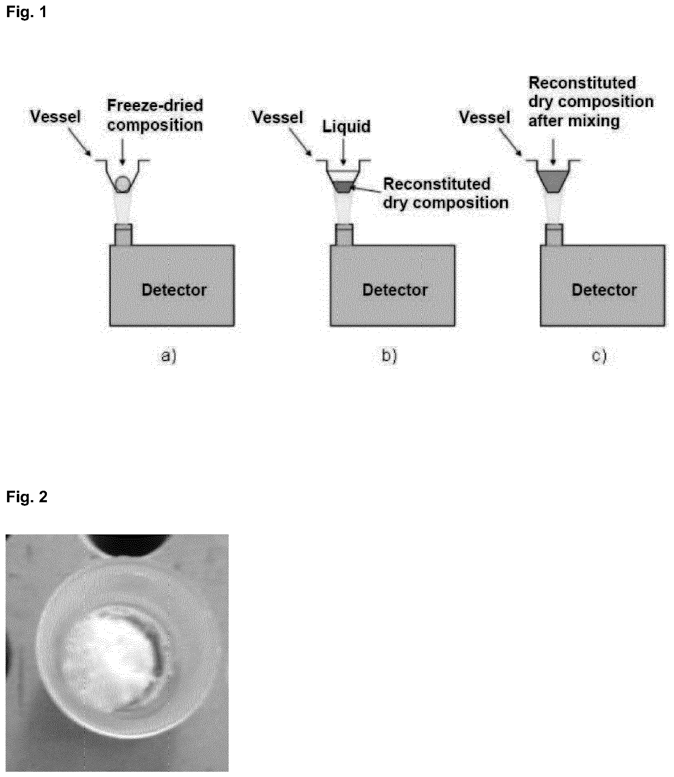

**FIG. 1** shows the characteristics of lyophilised reagents during reconstitution. The inventors found that when working with lyophilised reagents, they often fail to evenly distribute during reconstitution but rather remain concentrated at the bottom of the reaction vessel. This poses a problem, because these reaction reagents that are concentrated at the bottom might not be fully available for the chemical and/or biotechnological method. Thus, respectively concentrated reconstituted reagents might disturb the subsequent reaction and thus, can falsify the results. Therefore, to avoid such an uneven distribution of reconstituted reagents, mixing is required. However, mixing might not be successful and furthermore, errors in the system can also have the effect that no liquid is added for reconstitution and/or that not the right amount of liquid is added. In order to detect these different phenomena and to accurately monitor that liquid was correctly added for reconstitution and furthermore, to control that the dissolved reagents are distributed evenly, the present invention teaches to incorporate a control dye, in particular a fluorescent dye, into the dry composition of reagents. This can be achieved by adding the fluorescent dye to the soluble reagents prior to lyophilisation. The concept and differences between the different states of the freeze-dried reagents are illustrated in Fig. 1. Initially, the lyophilised reagents are provided as dried matter (see Fig. 1 a). Freeze-dried reagents comprising a fluorescent dye have a very high fluorescence and thus can be clearly distinguished from samples, to which liquid has been added for reconstitution. Upon contacting the lyophilised reagents with a liquid, the reagents are dissolved in the liquid but concentrate at the bottom of the reaction vessel (see Fig. 1 b). At the same time the fluorescence increases compared to a standard because of the high local fluorescence concentration. After mixing and homogenously distributing the reconstituted reagents, the fluorescence intensity decreases again, due to the homogenous distribution of the fluorescent dye within the solution (see Fig. 1 c). Thus, the method according to the present invention allows to clearly distinguish between these different scenarios, thereby providing a valuable control to detect variations/errors that might originate from the reconstitution process.
**FIG. 2** is a photograph of a lyophilised PCR-mastermix in an amplification chamber.
**FIG. 3** is a diagram showing the fluorescence-based detection of lyophilised reagents during reconstitution.
**FIG. 4** is a diagram showing the fluorescence-based detection of lyophilised PCR-mastermix reconstituted in diverse volumina.

### EXAMPLES

### Example 1

In this experiment 50µl of a PCR-mastermix comprising a fluorescent dye (rhodamine B) were transferred to several amplification chambers and lyophilised (see fig. 2).

Following lyophilisation, the fluorescence detection module was started. First, the fluorescence was detected for the references "no contents", "empty amplification chamber", "amplification chamber with RNase-free water" and "amplification chamber with liquid PCR mastermix".

Then, an amplification chamber comprising a lyophilised PCR mastermix was inserted into the module and the fluorescence was detected. Afterwards, water was added to the lyophilised reagents (no mixing), and the fluorescence was again detected. Finally, the sample was thoroughly mixed and the fluorescence was once more detected.

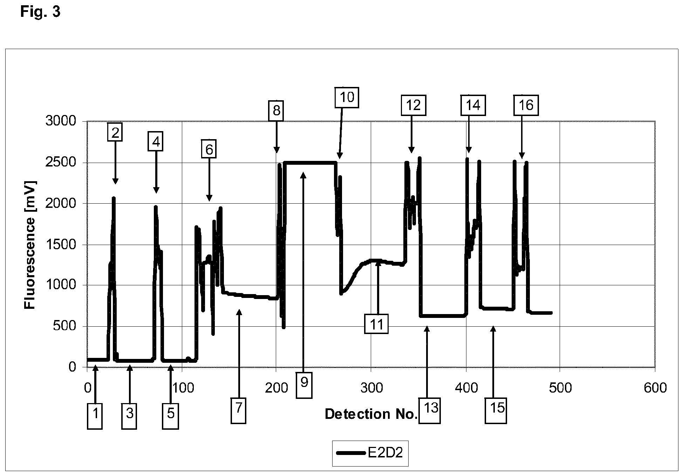

All of the above conditions tested were unequivocally differentiated using the teachings of the present invention by means of the corresponding fluorescence signal (see fig. 3).

The high peaks at positions 2, 4, 6, 8, 10, 12, 14 and 16 in Fig. 3 resulted from opening the lid of the detection module for changing or processing the amplification chambers and thus, are artificial. Incoming light reached the detector, thereby increasing the detection signal. The mark at position 9 corresponds to the detection of the lyophilised mastermix. The fluorescence intensity of rhodamine B in dried form was higher than the appointed detection limit (here 2500 mV). When dissolved in the reconstitution liquid, the fluorescence intensity decreased.

The other detection marks show the following:
1. detection of no contents (reference)
3. detection of empty amplification chamber (reference)
5. detection of amplification chamber with 50µl RNase-free water (reference)
7. detection of amplification chamber with 50µl liquid PCR mastermix (reference)
9. detection of amplification chamber with lyophilised PCR mastermix
11. detection of lyophilised mastermix during reconstitution with water
13. detection of lyophilised and reconstituted mastermix after first mixing
15. detection of lyophilised and reconstituted mastermix after second mixing

### Example 2

The aim of this experiment was to detect the filling quantity within an amplification chamber. It is also an important quality parameter to ensure that the pre-determined amount of reconstitution liquid is added to the free-dried reagent composition. If no sufficient liquid is added, the concentration of the reagents in the reconstituted solution could be too high, what poses again the risk that the subsequent reaction is falsified. If too much liquid is applied, the resulting concentration of reagents in the reconstituted solution can be too low. Thus, it is important to ensure that the reconstitution liquid is added in the pre-determined amount, potentially also considering tolerances.

Aliquoted samples of a lyophilised reagent (here: the PCR mastermix samples as described in example 1) were used, including a given amount of fluorescent dye (rhodamine B) in each amplification chamber. The fluorescence of the original fluorescent dye containing PCR mastermix was used as standard for comparison.

Prior to lyophilisation the original reagent liquid volume had been 50µl. Thus, the freeze-dried composition was reconstituted with the same amount of liquid. If this was not the case and instead a different reconstitution volume was used, then a change in the fluorescence signal is detectable according to the teachings of the present invention. Accordingly, too little reconstitution liquid would result in a higher fluorescence signal, while too much liquid would result in lower fluorescence as compared to the fluorescence signal measured for the PCR mastermix prior to lyophilisation.

To demonstrate the corresponding working principle of the method according to the present invention, the same experimental setup was used as in example 1. The detection was started and the module was loaded with an amplification chamber including a lyophilised mastermix and the fluorescence was detected. 50µl water was added and the fluorescence was again detected. Then, the sample was mixed and fluorescence was detected.

The amplification chamber was changed against a new chamber comprising a lyophilised mastermix and the fluorescence intensity was detected. Then, 25µl water (instead of 50µl) was added for reconstitution and the fluorescence was detected. The sample was mixed and fluorescence was detected one more time.

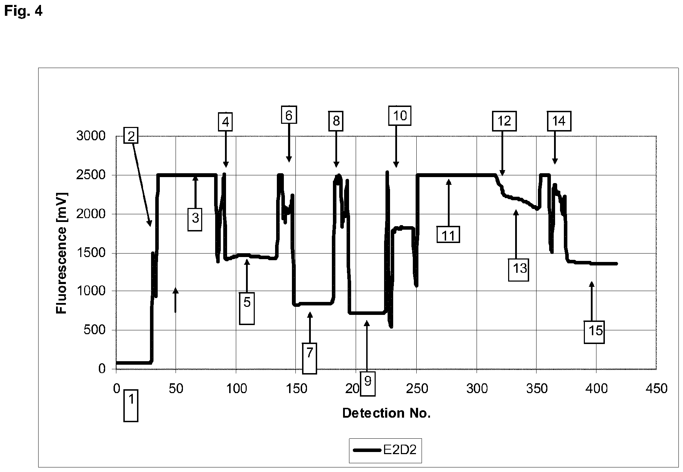

The results demonstrate that the fluorescence signal derived from the mastermix dissolved in 25µl water was double as compared to the (correct) 50µl mastermix sample (see fig. 4). Thus, the method according to the present invention can clearly distinguish between a sample that was reconstituted with the correct amount of liquid and a sample that was reconstituted with the wrong amount of liquid. Also all other tested conditions were unequivocally distinguished using the teachings of the present invention by means of the corresponding fluorescence signal.

The high peaks at positions 2, 4, 6, 8, 10, 12, 14 and 16 again result from opening the lid of the detection module for changing or processing the amplification chambers. The marks at position 3 and 11 correspond to the detection of the lyophilised mastermix. The fluorescence intensity of rhodamine B in dried form is higher than the detection limit (here 2500 mV). Upon reconstituting the freeze-dried mastermix, the fluorescence intensity decreases again. The other detection marks show the following:
1. detection of no contents (reference)
3. detection of amplification chamber with 50µl lyophilised PCR mastermix
5. detection of lyophilised mastermix during reconstitution with 50µl water
7. detection of lyophilised and with 50µl water reconstituted mastermix after first mixing
9. detection of lyophilised and with 50µl water reconstituted mastermix after second mixing
11. detection of amplification chamber with 50µl lyophilised PCR mastermix
13. detection of lyophilised mastermix during reconstitution with 25µl water
15. detection of lyophilised and with 25µl water reconstituted mastermix after first mixing

### Example 3

The aim of this experiment was to detect residual moisture within a lyophilised composition. Aliquoted samples of a lyophilised reagent (here: the PCR mastermix as described in example 1) were used, which comprised a given amount of fluorescent dye (rhodamine B) in each amplification chamber. Sample chambers were incubated in three different humidity areas (75%, 33% or 9% relative humidity), and fluorescence was detected after 1 day, 2 days and 8 days, respectively, in the dry compositions. The first measurement served to detect residual moisture in the dry product.

Afterwards, the samples were reconstituted with 50µl water to determine whether the samples had lost fluorescence intensity as compared to a liquid 50µl mastermix sample. Fluorescence was detected as described above.

The results detected in the dry composition are summarised in the subsequent table:

**Summary of the measured fluorescence [mV]**

| | **Reference** | **Day 1** | **Day 2** | **Day 8** |
|---|---|---|---|---|
| Tube 1_75% | 2165 | 1290 | 1272 | 1270 |
| Tube 2_75% | 1755 | 850 | 1000 | 903 |
| Tube 3_33% | 2040 | 1913 | 1789 | 1788 |
| Tube 4_33% | 1493 | 1313 | 1475 | 1390 |
| Tube 5_9% | 2100 | 2165 | 1940 | 1941 |
| Tube 6_9% | 2205 | 2242 | 1636 | 1710 |

The results demonstrate that samples with high residual moisture levels could be very well distinguished from dry samples based upon their fluorescence. Thus, this method allows to analyse whether a freeze-dried composition contains excess amounts of residual water and thus, might be compromised.

## Claims

1. A method of reconstituting a dry composition comprising at least one reagent for a chemical and/or biotechnological method, comprising the following steps:
a) obtaining a dry composition comprising at least one reagent and at least one control dye, preferably a fluorescent dye;
b) reconstituting the dry composition;
c) analyzing the reconstitution process by measuring at least one property of the control dye, preferably its fluorescence, during and/or after reconstitution.

2. A chemical and/or biotechnological method comprising the following steps:
a) obtaining a dry composition comprising at least one reagent and at least one control dye, preferably a fluorescent dye;
b) reconstituting the dry composition;
c) analysing the reconstitution process by measuring at least one property of the control dye, preferably its fluorescence, during and/or after reconstitution;
d) optionally adding further reagents and/or additives to the reconstituted composition;
e) performing the chemical and/or biotechnological method.

3. The method according to claim 1 or 2, **wherein** the control dye is a fluorescent dye.

4. The method according to one or more of claims 1 to 3, **wherein** a predefined volume of a liquid is added for reconstitution.

5. The method according to claim 3 or 4, having one or more of the following characteristics:
a) the measurement of the at least one property of the control dye is quantitative;
b) the amount of reconstituted reagent is quantified by detecting the fluorescence during and/or after reconstitution;
c) the homogeneity of the reconstituted dry composition is analysed by detecting the fluorescence during and/or after reconstitution;
d) it is analysed whether the dry composition is and/or has been reconstituted in the pre-determined amount of liquid by detecting the fluorescence during and/or after reconstitution;
e) variations in the filling quantity are determined by detecting the fluorescence during and/or after reconstitution;
f) the measured fluorescent signals are compared to at least one control and/or at least one standard; and/or
g) the at least one property of the control dye is also determined prior to reconstitution.

6. The method according to one or more of claims 3 to 5, **wherein** the fluorescent dye is selected from the group consisting of rhodamine dyes, SYBR green, pico green, auramine, benzanthrone, coelenterazine, coumarine, benzimide, DAPI, ethidium bromide, homidium bromide, euxanthic acid, firefly luciferin, fluoresceine, fluoresceine derivatives, carboxy fluorescein compounds, cyanine dyes, fluorescein isothiocyanate, perylene, 9,10-bis(phenylethynyl)anthracene, rubrene, stilbene, acridinium dyes, carbazol dyes, phenoxazine dyes, porphyrine dyes, polymethin dyes, BODIPY, Texas Red and TSQ.

7. The method according to one or more of claims 1 to 6, **wherein** the dry composition has one or more of the following characteristics:
a) it is a storable composition;
b) it is a freeze-dried composition;
c) it comprises at least some of the chemical and/or biochemical reagents necessary for conducting the intended chemical or biotechnological method;
d) it comprises at least one biological product;
e) it comprises at least some, preferably all of the chemical and/or biochemical reagents necessary for conducting an amplification reaction; and/or
f) it comprises one or more reagents selected from the group consisting of a polymerase, a reaction buffer suitable for performing an amplification reaction, primers and dNTPs.

8. The method according to one or more of claims 2 to 7, **wherein** the chemical and/or biotechnological method is an amplification reaction, preferably a PCR reaction.

9. The method according to one or more of claims 1 to 8, having one or more of the following characteristics:
a) a defined amount of the dry composition is provided in a vessel and is reconstituted with a pre-determined amount of a liquid; and/or
b) reconstitution is supported by mixing; and/or
c) the dry composition comprises magnetic material, preferably magnetic particles.

10. Use of at least one control dye, preferably at least one fluorescent dye, in a dry composition comprising at least one reagent suitable for a chemical and/or biotechnological method to analyse and/or monitor the reconstitution process.

11. The use according to claim 10 in a method according to one or more of claims 1 to 9.

12. A method for analysing residual moisture in a dry composition comprising at least one reagent suitable for performing a chemical and/or biotechnological method, wherein at least one control dye, preferably a fluorescent dye, is incorporated into the dry composition and wherein the residual moisture in the dry composition is determined by measuring in the dry composition at least one property of the control dye, preferably its fluorescence.

13. The method according to claim 12, **wherein** the control dye is a fluorescent dye, preferably selected from the group consisting of rhodamine dyes, SYBR green, pico green, auramine, benzanthrone, coelenterazine, coumarine, benzimide, DAPI, ethidium bromide, homidium bromide, euxanthic acid, firefly luciferin, fluoresceine, fluoresceine derivatives, carboxy fluorescein compounds, cyanine dyes, fluorescein isothiocyanate, perylene, 9,10-bis(phenylethynyl)anthracene, rubrene, stilbene, acridinium dyes, carbazol dyes, phenoxazine dyes, porphyrine dyes, polymethin dyes, BODIPY, Texas Red and TSQ.

14. The method according to claim 13, having one or more of the following characteristics:
a) the fluorescence of the dry composition is determined after manufacturing the dry composition;
b) the fluorescence of the dry composition is determined prior to reconstituting the dry composition in order to determine whether the dry composition was compromised during storage; and/or
c) the measured fluorescence is compared to a standard in order to determine whether the residual moisture is within a pre-defined range and/or above a pre-defined threshold.

15. Use of at least one fluorescent dye in a dry composition comprising at least one reagent for performing a chemical and/or biotechnological method for analysing residual moisture in said dry composition.

## Patentansprüche

1. Verfahren zum Rekonstituieren einer trockenen Zusammensetzung, die mindestens ein Reagenz für ein chemisches und/oder biotechnologisches Verfahren umfasst, das die folgenden Schritte umfasst:
a) Erhalt einer trockenen Zusammensetzung, die mindestens ein Reagenz und mindestens einen Kontrollfarbstoff, vorzugsweise einen Fluoreszenzfarbstoff, umfasst;
b) Rekonstituieren der trockenen Zusammensetzung;
c) Analysieren des Rekonstitutionsprozesses durch Messen mindestens einer Eigenschaft des Kontrollfarbstoffs, vorzugsweise seiner Fluoreszenz, während und/oder nach der Rekonstitution.

2. Chemisches und/oder biotechnologisches Verfahren, das die folgenden Schritte umfasst:
a) Erhalt einer trockenen Zusammensetzung, die mindestens ein Reagenz und mindestens einen Kontrollfarbstoff, vorzugsweise einen Fluoreszenzfarbstoff, umfasst;
b) Rekonstituieren der trockenen Zusammensetzung;
c) Analysieren des Rekonstitutionsprozesses durch Messen mindestens einer Eigenschaft des Kontrollfarbstoffs, vorzugsweise seiner Fluoreszenz, während und/oder nach der Rekonstitution;
d) optional Zugeben weiterer Reagenzien und/oder Additive zur rekonstituierten Zusammensetzung;
e) Durchführen des chemischen und/oder biotechnologischen Verfahrens.

3. Verfahren nach Anspruch 1 oder 2, wobei der Kontrollfarbstoff ein Fluoreszenzfarbstoff ist.

4. Verfahren nach einem oder mehreren der Ansprüche 1 bis 3, wobei ein vorgegebenes Volumen einer Flüssigkeit zur Rekonstitution zugegeben wird.

5. Verfahren nach Anspruch 3 oder 4, das eines oder mehrere der folgenden Merkmale aufweist:
a) die Messung der mindestens einen Eigenschaft des Kontrollfarbstoffs ist quantitativ;
b) die Menge des rekonstituierten Reagenzes wird quantifiziert, indem die Fluoreszenz während und/oder nach der Rekonstitution detektiert wird;
c) die Homogenität der rekonstituierten trockenen Zusammensetzung wird analysiert, indem die Fluoreszenz während und/oder nach der Rekonstitution detektiert wird;
d) es wird analysiert, ob die trockene Zusammensetzung in der vorbestimmten Flüssigkeitsmenge rekonstituiert wird und/oder wurde, indem die Fluoreszenz während und/oder nach der Rekonstitution detektiert wird;
e) Variationen der Füllmenge werden bestimmt, indem die Fluoreszenz während und/oder nach der Rekonstitution detektiert wird;
f) die gemessenen Fluoreszenzsignale werden mit mindestens einer Kontrolle und/oder mindestens einem Standard verglichen; und/oder
g) die mindestens eine Eigenschaft des Kontrollfarbstoffs wird auch vor der Rekonstitution bestimmt.

6. Verfahren nach einem oder mehreren der Ansprüche 3 bis 5, wobei der Fluoreszenzfarbstoff aus der Gruppe ausgewählt ist, die aus Rhodaminfarbstoffen, SYBR Green, Picogreen, Auramin, Benzanthron, Coelenterazin, Cumarin, Benzamid, DAPI, Ethidiumbromid, Homidiumbromid, Euxanthinsäure, Glühwürmchen-Luciferin, Fluoreszein, Fluoreszeinderivaten, Carboxy-Fluoreszein-Verbindungen, Cyaninfarbstoffen, Fluoreszeinisothiocyanat, Perylen, 9,10-Bis(phenylethynyl)anthrazen, Rubren, Stilben, Acridiniumfarbstoffen, Carbazolfarbstoffen, Phenoxazinfarbstoffen, Porphyrinfarbstoffen, Polymethinfarbstoffen, BODIPY, Texasrot und TSQ besteht.

7. Verfahren nach einem oder mehreren der Ansprüche 1 bis 6, wobei die trockene Zusammensetzung eines oder mehrere der folgenden Merkmale aufweist:
a) sie ist eine lagerfähige Zusammensetzung;
b) sie ist eine gefriergetrocknete Zusammensetzung;
c) sie umfasst mindestens einige der chemischen und/oder biochemischen Reagenzien, die notwendig sind, um das vorgesehene chemische oder biotechnologische Verfahren durchzuführen;
d) sie umfasst mindestens ein biologisches Erzeugnis;
e) sie umfasst mindestens einige, vorzugsweise alle der chemischen und/oder biochemischen Reagenzien, die notwendig sind, um eine Amplifikationsreaktion durchzuführen; und/oder
f) sie umfasst ein oder mehrere Reagenzien, die ausgewählt sind aus der Gruppe bestehend aus einer Polymerase, einem Reaktionspuffer, der für das Durchführen einer Amplifikationsreaktion geeignet ist, Primern und dNTPs.

8. Verfahren nach einem oder mehreren der Ansprüche 2 bis 7, wobei das chemische und/oder biotechnologische Verfahren eine Amplifikationsreaktion, vorzugsweise eine PCR-Reaktion, ist.

9. Verfahren nach einem oder mehreren der Ansprüche 1 bis 8, das eines oder mehrere der folgenden Merkmale aufweist:
a) eine definierte Menge der trockenen Zusammensetzung wird in einem Gefäß bereitgestellt und mit einer vorbestimmten Menge einer Flüssigkeit rekonstituiert; und/oder
b) die Rekonstitution wird durch Mischen unterstützt; und/oder
c) die trockene Zusammensetzung umfasst magnetisches Material, vorzugsweise magnetische Teilchen.

10. Verwendung von mindestens einem Kontrollfarbstoff, vorzugsweise mindestens einem Fluoreszenzfarbstoff, in einer trockenen Zusammensetzung, die mindestens ein Reagenz umfasst, das für ein chemisches und/oder biotechnologisches Verfahren geeignet ist, um den Rekonstitutionsprozess zu analysieren und/oder zu überwachen.

11. Verwendung nach Anspruch 10 in einem Verfahren nach einem oder mehreren der Ansprüche 1 bis 9.

12. Verfahren zum Analysieren von Restfeuchtigkeit in einer trockenen Zusammensetzung, die mindestens ein Reagenz umfasst, das für das Durchführen eines chemischen und/oder biotechnologischen Verfahrens geeignet ist, wobei mindestens ein Kontrollfarbstoff, vorzugsweise ein Fluoreszenzfarbstoff, in die trockene Zusammensetzung aufgenommen ist und wobei die Restfeuchtigkeit in der trockenen Zusammensetzung bestimmt wird, indem in der trockenen Zusammensetzung mindestens eine Eigenschaft des Kontrollfarbstoffs, vorzugsweise dessen Fluoreszenz, gemessen wird.

13. Verfahren nach Anspruch 12, wobei der Kontrollfarbstoff ein Fluoreszenzfarbstoff ist, der vorzugsweise aus der Gruppe ausgewählt ist, die aus Rhodaminfarbstoffen, SYBR Green, Picogreen, Auramin, Benzanthron, Coelenterazin, Cumarin, Benzamid, DAPI, Ethidiumbromid, Homidiumbromid, Euxanthinsäure, Glühwürmchen-Luciferin, Fluoreszein, Fluoreszeinderivaten, Carboxy-Fluoreszein-Verbindungen, Cyaninfarbstoffen, Fluoreszeinisothiocyanat, Perylen, 9,10-Bis(phenylethynyl)anthrazen, Rubren, Stilben, Acridiniumfarbstoffen, Carbazolfarbstoffen, Phenoxazinfarbstoffen, Porphyrinfarbstoffen, Polymethinfarbstoffen, BODIPY, Texasrot und TSQ besteht.

14. Verfahren nach Anspruch 13, das eines oder mehrere der folgenden Merkmale aufweist:
a) die Fluoreszenz der trockenen Zusammensetzung wird nach der Herstellung der trockenen Zusammensetzung bestimmt;
b) die Fluoreszenz der trockenen Zusammensetzung wird vor der Rekonstitution der trockenen Zusammensetzung bestimmt, um zu bestimmen, ob die trockene Zusammensetzung während der Lagerung beeinträchtigt wurde; und/oder
c) die gemessene Fluoreszenz wird mit einem Standard verglichen, um zu bestimmen, ob die Restfeuchtigkeit innerhalb eines vorgegebenen Bereichs und/oder über einem vorgegebenen Schwellenwert liegt.

15. Verwendung mindestens eines Fluoreszenzfarbstoffs in einer trockenen Zusammensetzung, die mindestens ein Reagenz zum Durchführen eines chemischen und/oder biotechnologischen Verfahrens umfasst, zum Analysieren der Restfeuchtigkeit in der trockenen Zusammensetzung.

## Revendications

1. Procédé de reconstitution d'une composition sèche comprenant au moins un réactif pour un procédé chimique et/ou biotechnologique, comprenant les étapes suivantes :
a) obtenir une composition sèche comprenant au moins un réactif et au moins un colorant de contrôle, de préférence un colorant fluorescent ;
b) reconstituer la composition sèche ;
c) analyser le processus de reconstitution en mesurant au moins une propriété du colorant de contrôle, de préférence sa fluorescence, pendant et/ou après reconstitution.

2. Procédé chimique et/ou biotechnologique comprenant les étapes suivantes :
a) obtenir une composition sèche comprenant au moins un réactif et au moins un colorant de contrôle, de préférence un colorant fluorescent ;
b) reconstituer la composition sèche ;
c) analyser le processus de reconstitution en mesurant au moins une propriété du colorant de contrôle, de préférence sa fluorescence, pendant et/ou après reconstitution ;
d) optionnellement ajouter d'autres réactifs et/ou additifs à la composition reconstituée ;
e) réaliser le procédé chimique et/ou biotechnologique.

3. Procédé selon la revendication 1 ou 2, **dans lequel** le colorant de contrôle est un colorant fluorescent.

4. Procédé selon une ou plusieurs des revendications 1 à 3, **dans lequel** un volume prédéfini d'un liquide est ajouté pour reconstitution.

5. Procédé selon la revendication 3 ou 4, présentant une ou plusieurs des caractéristiques suivantes :
a) la mesure de la au moins une propriété du colorant de contrôle est quantitative ;
b) la quantité de réactif reconstitué est quantifiée en détectant la fluorescence pendant et/ou après reconstitution ;
c) l'homogénéité de la composition sèche reconstituée est analysée en détectant la fluorescence pendant et/ou après reconstitution ;
d) il est analysé si la composition sèche est et/ou a été reconstituée dans la quantité prédéterminée de liquide en détectant la fluorescence pendant et/ou après reconstitution ;
e) des variations de la quantité de remplissage sont déterminées en détectant la fluorescence pendant et/ou après reconstitution ;
f) les signaux fluorescents mesurés sont comparés à au moins un témoin et/ou au moins un étalon ; et/ou
g) la au moins une propriété du colorant de contrôle est également déterminée avant reconstitution.

6. Procédé selon une ou plusieurs des revendications 3 à 5, **dans lequel** le colorant fluorescent est sélectionné parmi le groupe constitué de colorants de rhodamine, du vert SYBR, du PicoGreen, de l'auramine, de la benzanthrone, de la coelentérazine, de la coumarine, du benzimide, du DAPI, du bromure d'éthidium, du bromure d'homidium, de l'acide euxanthique, de la luciférine de luciole, de la fluorescéine, de dérivés de fluorescéine, de composés de carboxyfluorescéine, de colorants de cyanine, de l'isothiocyanate de fluorescéine, du pérylène, du 9,10-bis(phényléthynyl)anthracène, du rubrène, du stilbène, de colorants d'acridinium, de colorants de carbazole, de colorants de phénoxazine, de colorants de porphyrine, de colorants de polyméthine, du BODIPY, du Texas Red et du TSQ.

7. Procédé selon une ou plusieurs des revendications 1 à 6, **dans lequel** la composition sèche présente une ou plusieurs des caractéristiques suivantes :
a) il s'agit d'une composition pouvant être stockée ;
b) il s'agit d'une composition lyophilisée ;
c) elle comprend au moins certains des réactifs chimiques et/ou biochimiques nécessaires pour mener le procédé chimique ou biotechnologique prévu ;
d) elle comprend au moins un produit biologique ;
e) elle comprend au moins certains, de préférence la totalité des réactifs chimiques et/ou biochimiques nécessaires pour mener une réaction d'amplification ; et/ou
f) elle comprend un ou plusieurs réactifs sélectionnés parmi le groupe constitué d'une polymérase, d'un tampon réactionnel adapté pour effectuer une réaction d'amplification, d'amorces et de dNTP.

8. Procédé selon une ou plusieurs des revendications 2 à 7, **dans lequel** le procédé chimique et/ou biotechnologique est une réaction d'amplification, de préférence une réaction PCR.

9. Procédé selon une ou plusieurs des revendications 1 à 8, présentant une ou plusieurs des caractéristiques suivantes :
a) une quantité définie de la composition sèche est introduite dans un récipient et est reconstituée avec une quantité prédéterminée d'un liquide ; et/ou
b) une reconstitution est supportée par mélange ; et/ou
c) la composition sèche comprend un matériau magnétique, de préférence des particules magnétiques.

10. Utilisation d'au moins un colorant de contrôle, de préférence au moins un colorant fluorescent, dans une composition sèche comprenant au moins un réactif adapté à un procédé chimique et/ou biotechnologique pour analyser et/ou surveiller le processus de reconstitution.

11. Utilisation selon la revendication 10 dans un procédé selon une ou plusieurs des revendications 1 à 9.

12. Procédé d'analyse d'humidité résiduelle dans une composition sèche comprenant au moins un réactif adapté pour effectuer un procédé chimique et/ou biotechnologique, dans lequel au moins un colorant de contrôle, de préférence un colorant fluorescent, est incorporé dans la composition sèche et où l'humidité résiduelle dans la composition sèche est déterminée en mesurant dans la composition sèche au moins une propriété du colorant de contrôle, de préférence sa fluorescence.

13. Procédé selon la revendication 12, **dans lequel** le colorant de contrôle est un colorant fluorescent, de préférence sélectionné parmi le groupe constitué de colorants de rhodamine, du vert SYBR, du PicoGreen, de l'auramine, de la benzanthrone, de la coelentérazine, de la coumarine, du benzimide, du DAPI, du bromure d'éthidium, du bromure d'homidium, de l'acide euxanthique, de la luciférine de luciole, de la fluorescéine, de dérivés de fluorescéine, de composés de carboxyfluorescéine, de colorants de cyanine, de l'isothiocyanate de fluorescéine, du pérylène, du 9,10-bis(phényléthynyl)anthracène, du rubrène, du stilbène, de colorants d'acridinium, de colorants de carbazole, de colorants de phénoxazine, de colorants de porphyrine, de colorants de polyméthine, du BODIPY, du Texas Red et du TSQ.

14. Procédé selon la revendication 13, présentant une ou plusieurs des caractéristiques suivantes :
a) la fluorescence de la composition sèche est déterminée après fabrication de la composition sèche ;
b) la fluorescence de la composition sèche est déterminée avant reconstitution de la composition sèche de manière à déterminer si la composition sèche s'est dégradée pendant le stockage ; et/ou
c) la fluorescence mesurée est comparée à un étalon de manière à déterminer si l'humidité résiduelle se situe dans une plage prédéfinie et/ou au-dessus d'un seuil prédéfini.

15. Utilisation d'au moins un colorant fluorescent dans une composition sèche comprenant au moins un réactif pour effectuer un procédé chimique et/ou biotechnologique en vue d'analyser l'humidité résiduelle dans ladite composition sèche.
